# EUROPEAN PATENT APPLICATION

(11) **EP 2 254 033 A1**
(43) Date of publication of application: **24.11.2010**
(21) Application number: 09177852.2
(22) Date of filing: 03.12.2009
(51) Int. Cl.: G06F 3/048, A61B 8/00

(54) **Ultrasound diagnosis apparatus using touch interaction**

(30) Priority: 22.05.2009 KR 20090044949
(71) Applicant: MEDISON CO., LTD., Gangwon-do 250-875 (KR)
(72) Inventor: Shin, Soo-Hwan, 137-061, Seoul (KR)
(74) Representative: Schmid, Wolfgang

(57) **Abstract**

Disclosed is an ultrasound diagnosis apparatus utilizing a touch interaction. The ultrasound diagnosis apparatus may include an ultrasound image generating unit to generate an ultrasound image, a touch input unit to receive a touch interaction inputted from a user, a sensor to sense the touch interaction from the touch input unit, a controller to control an operation for processing the ultrasound image according to the sensed touch interaction, and a display unit to display the ultrasound image according to the controlling of the controller. Here, the touch interaction includes at least one touch operation that is capable of a multi-touch with respect to the touch input unit.

## Description

### BACKGROUND

### 1. Field

The present invention relates to an ultrasound diagnosis apparatus using a touch interaction, and more particularly, to an ultrasound diagnosis apparatus that may reduce a number of required buttons due to a touch interaction and may reduce a manipulation or movement by a user.

### 2. Description of the Related Art

Generally, an ultrasound diagnosis apparatus includes a probe having a broadband transducer to transmit and receive an ultrasound signal. When the transducer is electrically stimulated, the ultrasound diagnosis apparatus generates the ultrasound signal and transmits the generated ultrasound signal to a human body. The ultrasound signal transmitted to the human body is reflected from a boundary of a tissue inside the human body and an ultrasound echo signal transferred to the transducer from the boundary of the tissue inside the human body is converted into an electrical signal. The ultrasound diagnosis apparatus may perform amplification and signal-process with respect to the converted electrical signal to generate ultrasound image data for an image of the tissue, and may display the generated ultrasound image.

An important matter for a recently released portable ultrasound diagnosis apparatus is making the apparatus as small and as light as possible while maintaining an existing function and efficiency of an existing card-based apparatus. However, a conventional portable ultrasound diagnosis apparatus uses a one to two inch trackball, and thus, there is a limitation to decreasing the size due to a size of the trackball.

Also, the conventional portable ultrasound diagnosis apparatus is manufactured to be small and has an insufficient amount of space to arrange an input apparatus, such as a button and the like, and thus, low priority functions are removed and only a predetermined small number of buttons are provided.

### SUMMARY

An aspect of the present invention provides an ultrasound diagnosis apparatus that may provide various functions by using a touch interaction and may decrease a number of required buttons, thereby decreasing a size of the ultrasound diagnosis apparatus.

Another aspect of the present invention provides an ultrasound diagnosis apparatus that may decrease an operation or a movement to perform a function by utilizing a touch interaction.

According to example embodiments, there may be provided an ultrasound diagnosis apparatus, including an ultrasound image generating unit to generate an ultrasound image, a touch input unit to receive a touch interaction inputted from a user, a sensor to sense the touch interaction from the touch input unit, a controller to control an operation for processing the ultrasound image according to the sensed touch interaction, and a display unit to display the ultrasound image according to the controlling of the controller. Here, the touch interaction includes at least one touch operation that is capable of multi-touching with respect to the touch input unit.

Additional aspects and/or advantages will be set forth in part in the description which follows and, in part, will be apparent from the description, or may be learned by practice of the embodiments.

### Effect

According to the present invention, there may be provided an ultrasound diagnosis apparatus that may provide various functions by using a touch interaction and may decreases a number of required buttons, thereby decreasing a size of a the ultrasound diagnosis apparatus.

According to the present invention, there may be provided an ultrasound diagnosis apparatus that may decrease an operation or a movement to perform a function by utilizing a touch interaction.

### BRIEF DESCRIPTION OF THE DRAWINGS

These and/or other aspects and advantages will become apparent and more readily appreciated from the following description of the embodiments, taken in conjunction with the accompanying drawings of which:
- FIG. 1: is a diagram illustrating a configuration of an ultrasound diagnosis apparatus according to an exemplary embodiment of the present invention;
- FIG. 2: is a diagram illustrating a drag operation as an example of a touch interaction according to an exemplary embodiment the present invention;
- FIG. 3: is a diagram illustrating a tap operation as an example of a touch interaction according to an exemplary embodiment the present invention;
- FIG. 4: is a diagram illustrating a circular-drag operation as an example of a touch interaction according to an exemplary embodiment the present invention;
- FIG. 5: is a diagram illustrating a long tap operation as an example of a touch interaction according to an exemplary embodiment the present invention;
- FIG. 6: is a diagram illustrating a flick operation as an example of a touch interaction according to an exemplary embodiment the present invention;
- FIG. 7: is a diagram illustrating an item changed by a touch interaction, in an M mode, according to an exemplary embodiment the present invention;
- FIG. 8: is a diagram illustrating an item changed by a touch interaction, in a PW mode, according to an exemplary embodiment the present invention; and
- FIG. 9: is a diagram illustrating an item changed by a touch interaction, in a color (C)/Power Doppler (PD) mode or a 3D standby state, according to an exemplary embodiment the present invention.

### DETAILED DESCRIPTION

Reference will now be made in detail to example embodiments, examples of which are illustrated in the accompanying drawings, wherein like reference numerals refer to the like elements throughout. An ultrasound diagnosis apparatus utilizing a touch interaction is described below to explain the present disclosure by referring to the figures.

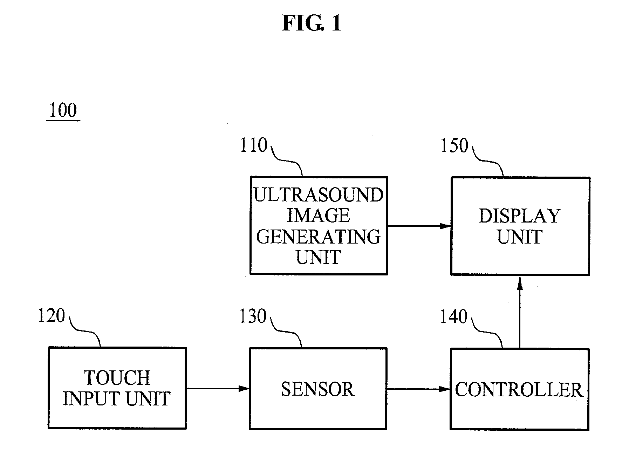

FIG. 1 is a diagram illustrating a configuration of an ultrasound diagnosis apparatus according to an exemplary embodiment of the present invention.

Referring to FIG. 1, the ultrasound diagnosis apparatus 100 according to an exemplary embodiment of the present invention includes an ultrasound image generating unit 110, a touch input unit 120, a sensor 130, a controller 140, and a display unit 150.

The ultrasound image generating unit 110 may generate an ultrasound image. That is, in the ultrasound diagnosis apparatus 100, the ultrasound image generating unit 110 may generate an ultrasound image that is a result of an ultrasound diagnosis with respect to a patient.

The touch input unit 120 may receive a touch interaction inputted from the user. In the present invention, the touch interaction may include at least one touch operation including a multi-touch. The touch interaction may include various operations, such as a tap, a double tap, a drag, a long tap, a flick, a multi-touch, and the like. The tap may be an operation of clicking (touching), the double tap may be an operation of consecutively clicking (touching) twice, and the drag may be an operation that the user touches on the touch input unit 120 and drags his/her finger up and down or left and right at a relatively constant speed. The long tap may be an operation of continuously pushing on one point, the flick is a fast linear movement related to a predetermined command, and the multi-touch is an operation of simultaneously pushing on two or more touch points.

As an example, the touch input unit 120, which is a means for inputting a desired function to the ultrasound diagnosis apparatus 100 through a user's touch operation, instead of an operation input button, may be embodied by various touch input means, as well as a touch pad.

Accordingly, the present invention may provide the ultrasound diagnosis apparatus 100 providing various required functions by utilizing the touch interaction and decreasing a size of a product by decreasing the number of the required buttons.

The sensor 130 may sense the touch interaction of user inputted from the touch input unit 120. That is, the sensor 130 may sense the touch interaction of the user inputted through the touch input unit 120.

The controller 140 may control an operation for processing the ultrasound image according to the sensed touch interaction. That is, the controller 140 may control an operation for changing an image or a state with respect to the ultrasound image according to the sensed touch interaction.

As an example, when the touch interaction is sensed while the ultrasound image is scanned, the controller 140 may control the image or the state with respect to the ultrasound image to change according to the sensed touch interaction.

The display unit 150 may display the ultrasound image according to controlling of the controller 140. That is, the display unit 150 may display the ultrasound image of which an image or a state is changed, according to the controlling of the controller 140.

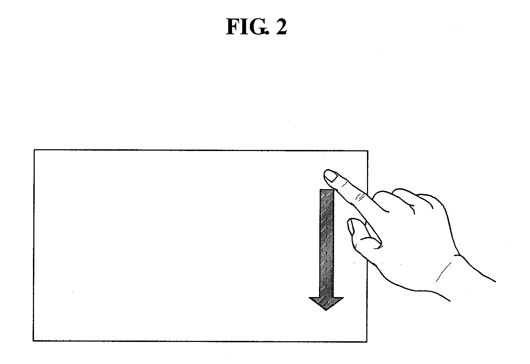

FIG. 2 is a diagram illustrating a drag operation as an example of a touch interaction according to an exemplary embodiment the present invention.

Referring to FIG. 2, the touch input unit 120 may receive an operation inputted from a user, the operation being dragging up to down at a regular speed, while the ultrasound image is scanned. The sensor 130 may sense the operation that drags up to down as illustrated in FIG. 2, as the touch interaction. The controller 140 may control a depth with respect to an image of the ultrasound image to be adjusted, when the touch interaction sensed while the ultrasound image is scanned is the operation of dragging up to down.

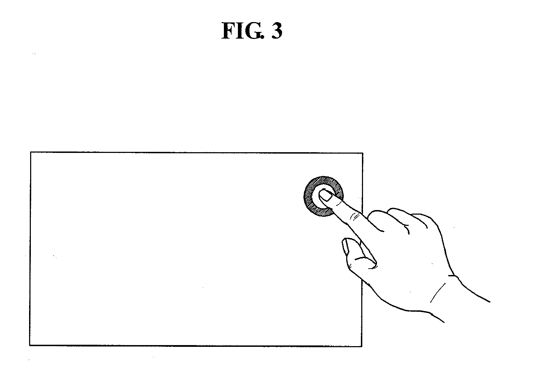

FIG. 3 is a diagram illustrating a tap operation as an example of a touch interaction according to an exemplary embodiment the present invention.

Referring to FIG. 3, the touch input unit 120 may receive a tap operation inputted from a user, the tap operation being clicking (touching) one time, while the ultrasound image is scanned. The sensor 130 may sense the tap operation that clicks one time, the tap operation being inputted from the user as illustrated in FIG. 3, as the touch interaction. The controller 140 may control a state of the ultrasound image to change into a freeze state where a last image of the ultrasound image is displayed, when the touch interaction sensed while the ultrasound image is scanned is the operation of clicking one time.

Also, the touch input unit 120 may receive an operation of dragging two points, the operation being inputted from the user, while the ultrasound image is scanned. The sensor 130 may sense, from the touch input unit 120, the operation of the dragging the two points, as the touch interaction. The controller 140 may control a function of a zoom with respect to the ultrasound image, when the touch interaction sensed while the ultrasound image is scanned is the operation of dragging two points.

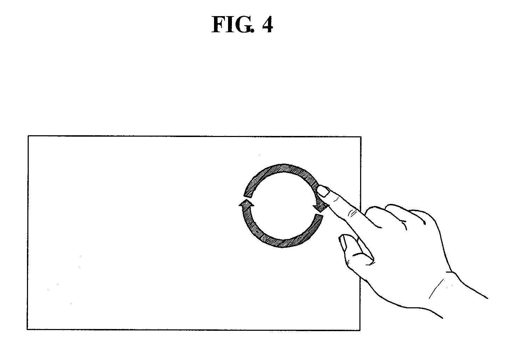

FIG. 4 is a diagram illustrating a circular-drag operation as an example of a touch interaction according to an exemplary embodiment the present invention.

Referring to FIG. 4, the touch input unit 120 may receive a circular-drag operation inputted from a user while the ultrasound image is scanned. The sensor 130 may sense, from the touch input unit 120, the circular-drag operation of FIG. 4, as the touch interaction. The controller 140 may control the ultrasound image to rotate, when the touch interaction sensed while the ultrasound image is scanned is the circular-drag operation.

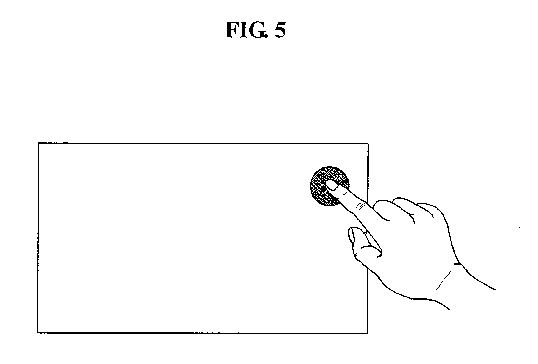

FIG. 5 is a diagram illustrating a long tap operation as an example of a touch interaction according to an exemplary embodiment the present invention.

Referring to FIG. 5, the touch input unit 120 may receive a long tap operation of continuously pushing on one point, the operation being inputted from a user, while the ultrasound image is scanned. The sensor 130 may sense, from the touch input unit 120, the long tap operation of continuously pushing on the one point as illustrated in FIG. 5, as the touch interaction. The controller 140 may control a Cine image with respect to the ultrasound image to be stored, when the touch interaction sensed while the ultrasound image is scanned is the operation that continuously pushes on the one point.

Also, the controller 140 may control a state of the ultrasound image to change into a live state, when the sensed touch interaction is clicking one time in the freeze state.

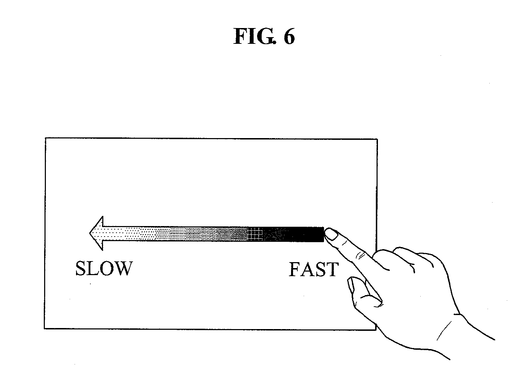

FIG. 6 is a diagram illustrating a flick operation as an example of a touch interaction according to an exemplary embodiment the present invention.

Referring to FIG. 6, the touch input unit 120 may receive a flick operation that is a linear movement in the freeze state, the flick operation being inputted from a user and a speed of the linear movement to the left and a speed of the linear movement to the right being different from each other. The sensor 130 may sense, from the touch input unit 120, the linear movement that moves from a right to a left with a different speed from a fast speed to a slow speed, as the touch interaction. The controller 140 may control a movement of an image search bar in the freeze state, when the sensed touch interaction is the linear movement moving from the right to the left with different speed.

Also, the controller 140 may control a thumbnail page to convert, when the sensed touch interaction is a linear movement with respect to the thumbnail in the freeze state, a speed of the linear movement in a left and a speed of the linear movement in a right being different from each other or a speed of the linear movement upward and a speed of the linear movement downward being different from each other. In this instance, a direction of the up and down or a direction of the left and right may be different based on an arranged direction of the thumbnail

Also, the controller 140 may control an image with respect to the ultrasound image to be stored, when the sensed touch interaction is an operation of continuously pushes on one point in the freeze state.

Also, the controller 140 may control a display state of an ultrasound image corresponding to the sensed touch interaction to change for each mode for displaying the ultrasound image.

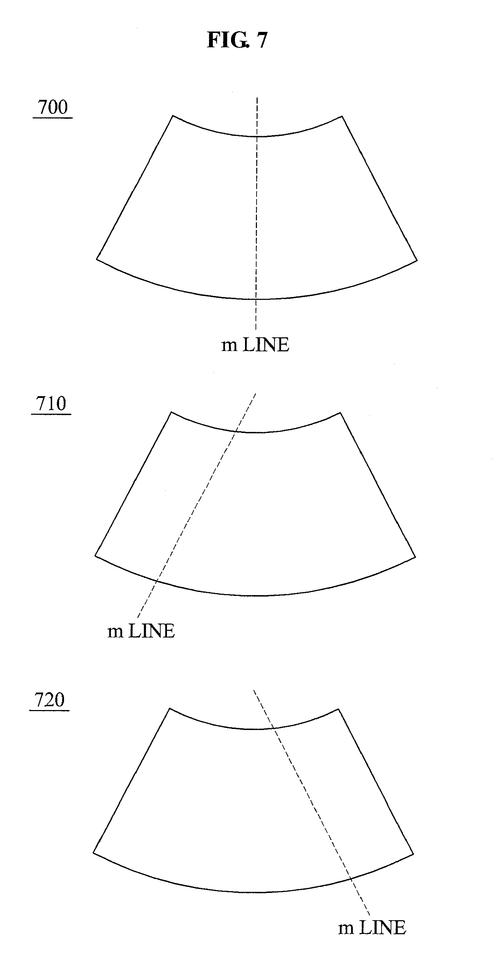

FIG. 7 is a diagram illustrating an item changed by a touch interaction, in an M mode, according to an exemplary embodiment the present invention.

Referring to FIG. 7, the controller 140 may control an m-line to move, when the mode for displaying the ultrasound image is an M mode and the sensed touch interaction is an operation of dragging left and right. As an example, the controller 140 may control the m-line located in a center of a reference screen 700 to move to the left or to the right, such as a first changed screen 710 and a second changed screen 720, when the mode for displaying the ultrasound image is the M mode and the sensed touch interaction is the operation of dragging left and right.

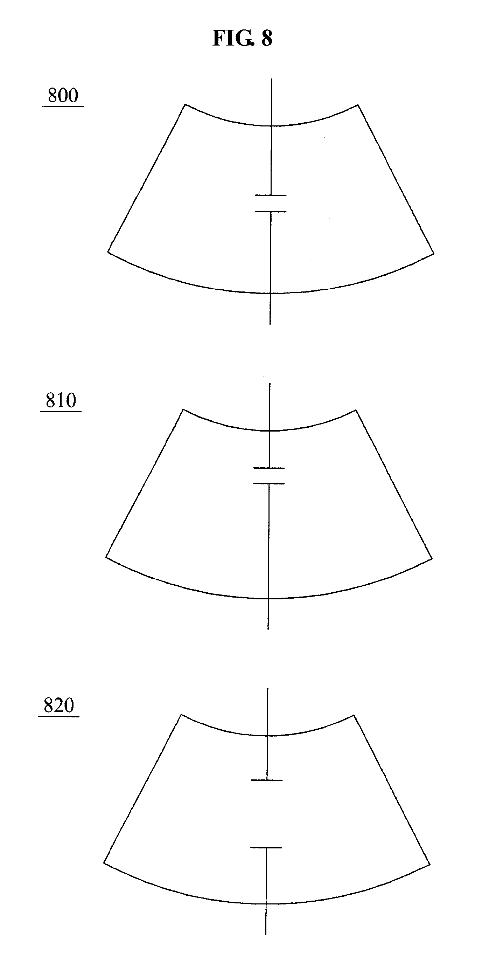

FIG. 8 is a diagram illustrating an item changed by a touch interaction, in a PW mode, according to an exemplary embodiment the present invention.

Referring to FIG. 8, the controller 140 controls a location of a sample volume (SV) to move when the mode for displaying the ultrasound image is a PW mode and the sensed touch interaction is an operation of up-down or left-right dragging. As an example, when the mode for displaying the ultrasound image is the PW mode and the sensed touch interaction is an operation of dragging upward, the controller 140 may control the SV located in a middle of a reference screen 800 to move upward, such as a first changed screen 810.

Also, when the mode for displaying the ultrasound image is the PW mode and the sensed touch interaction is an operation of simultaneously touching two or more points, the controller 140 may controls a size of the SV. As an example, when the mode for displaying the ultrasound image is the PW mode, and the sensed touch interaction is the operation of simultaneously touching two or more points, the controller 140 may control a size of the SV of the reference screen 800 to be magnified, such as a second changed screen 820.

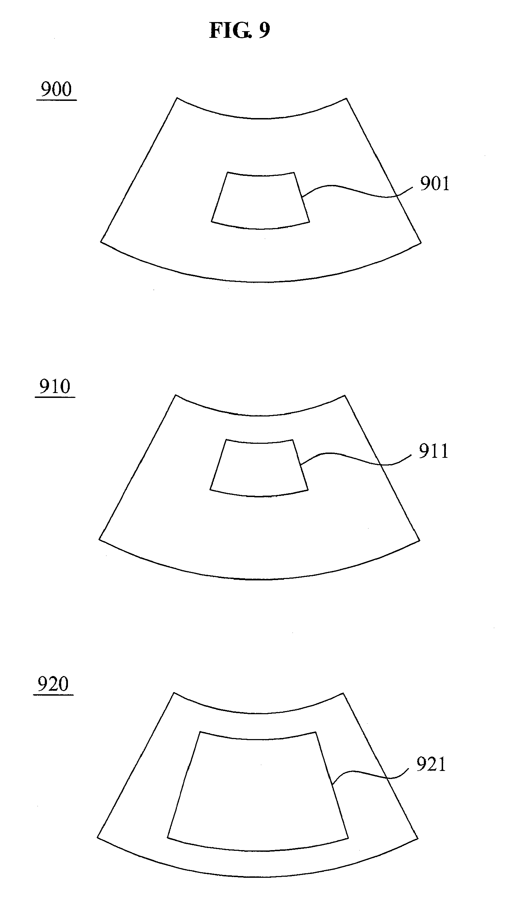

FIG. 9 is a diagram illustrating an item changed by a touch interaction, in a color (C)/Power Doppler (PD) mode or a 3D standby state, according to an exemplary embodiment the present invention.

Referring to FIG. 9, when the mode for displaying the ultrasound image is a C mode and the sensed touch interaction is an operation of up-down or left-right dragging, the controller 140 may control a location of a region of interest (ROI) to change. As an example, when the mode for displaying the ultrasound image is the C mode, and the sensed touch interaction is the operation of dragging upward, the controller 140 may control a location of a reference ROI 901 in a reference screen 900 to move to a location of a first ROI 911 of a first changed screen 910.

When the mode for displaying the ultrasound image is the C, and the sensed touch interaction is the operation of simultaneously touching two or more points, the controller 140 may control a size of the ROI. As an example, when the sensed touch interaction that is the operation of simultaneously touching two or more points is greater than a reference amount, the controller 140 may control the size of the reference ROI 901 of the reference screen 900 to be magnified such as a second ROI 921 of a second changed screen 920.

When the mode for displaying the ultrasound image is a PD mode and the sensed touch interaction is an operation of up-down or left-right dragging, the controller 140 may control the location of the ROI to change. Also, when the mode for displaying the ultrasound image is the PD mode and the sensed touch interaction is an operation of simultaneously touching two or more points, the controller 140 may control the size of the ROI. That is, the controller 140 either controls a location of an ROI to change, when the mode for displaying the ultrasound image is a Power Doppler (PD) mode and the sensed touch interaction is an operation of up-down or left-right dragging, or controls a size of an ROI to change when the sensed touch interaction is an operation of simultaneously touching two or more points.

When the mode for displaying the ultrasound image is a three-dimensional (3D) standby state and the sensed touch interaction is an operation of up-down or left-right dragging, the controller 140 may control the location of the ROI to change. Also, the controller 140 may control the size of the ROI to change when the sensed touch interaction is an operation of simultaneously touching two or more points. That is, the controller 140 either controls a location of a ROI to be change when the mode for displaying the ultrasound image is a three-dimensional (3D) standby state and the sensed touch interaction is an operation of up-down or left-right dragging, or controls a size of the ROI to change when the sensed touch interaction is an operation of simultaneously touching two or more points.

As described above, the present invention may provide an ultrasound diagnosis apparatus that decreases an operation or a movement to perform a function for each mode by utilizing various touch interaction.

Although a few example embodiments have been shown and described, it would be appreciated by those skilled in the art that changes may be made in these example embodiments without departing from the principles and spirit of the invention, the scope of which is defined in the claims and their equivalents.

## Claims

1. An ultrasound diagnosis apparatus, comprising:
an ultrasound image generating unit to generate an ultrasound image;
a touch input unit to receive a touch interaction inputted from a user;
a sensor to sense the touch interaction from the touch input unit;
a controller to control an operation for processing the ultrasound image according to the sensed touch interaction; and
a display unit to display the ultrasound image according to the controlling of the controller
wherein, the touch interaction includes at least one touch operation that is capable of multi-touching with respect to the touch input unit.

2. The apparatus of claim 1, wherein, when the touch interaction is sensed while the ultrasound image is scanned, the controller controls an image or a state with respect to the ultrasound image to change according to the sensed touch interaction.

3. The apparatus of claim 2, wherein the controller controls a depth with respect to the image of the ultrasound image to be adjusted, when the touch interaction sensed while the ultrasound image is scanned is an operation of dragging up to down.

4. The apparatus of claim 2, wherein the controller controls a state of the ultrasound image to change into a freeze state where a last image of the ultrasound image is display, when the touch interaction sensed while the ultrasound image is scanned is an operation of clicking one time.

5. The apparatus of claim 2, wherein the controller controls a zoom function with respect to the ultrasound image, when the touch interaction sensed while the ultrasound image is scanned is an operation of dragging two points.

6. The apparatus of claim 2, wherein the controller controls the ultrasound image to rotate when the touch interaction is sensed while the ultrasound image is scanned is an operation of circular-dragging.

7. The apparatus of claim 2, wherein the controller controls a Cine image with respect to the ultrasound image to be stored, when the touch interaction sensed while the ultrasound image is scanned is an operation of continuously pushing on one point.

8. The apparatus of claim 4, wherein the controller controls the state of the ultrasound image to change into a live state where the ultrasound image is obtained, when the sensed touch interaction is the one-click in the freeze state.

9. The apparatus of claim 4, wherein the controller controls a movement of an image search bar when the sensed touch interaction is a linear movement in the freeze state, a speed of the linear movement in a left and a speed of the linear movement in a right being different from each other.

10. The apparatus of claim 4, wherein the controller controls a thumbnail page to be converted, when the sensed touch interaction is a linear movement with respect to a thumbnail in the freeze state, a speed of the linear movement to the left and a speed of the linear movement to the right being different from each other or a speed of the linear movement upward and a speed of the linear movement downward being different from each other.

11. The apparatus of claim 4, wherein the controller controls the image with respect to the ultrasound image to be stored, when the sensed touch interaction is an operation of continuously pushing on one point in the freeze state.

12. The apparatus of claim 1, wherein the controller controls a display state of the ultrasound corresponding to the sensed touch interaction to change for each mode for displaying the ultrasound image.

13. The apparatus of claim 12, wherein the controller controls an m-line to move, when the mode for displaying the ultrasound image is an M mode and the sensed touch interaction is an operation of left-right dragging.

14. The apparatus of claim 12, wherein, the controller either controls a location of a sample volume (SV) to move when the mode for displaying the ultrasound image is a PW mode and the sensed touch interaction is an operation of up-down or left-right dragging, or controls a size of the SV to change when the sensed touch interaction is an operation of simultaneously touching two or more points.

15. The apparatus of claim 12, wherein the controller either controls a location of a region of interest (ROI) to change when the mode for displaying the ultrasound image is a color (C) mode and the sensed touch interaction is an operation of up-down or left-right dragging, or controls a size of the ROI to change, when the sensed touch interaction is an operation of simultaneously touching two or more points.
